Europäisches Patentamt

**European Patent Office**

**Office européen des brevets**

(11) Publication number: **0 156 296**
A1

# EUROPEAN PATENT APPLICATION

(21) Application number: 85103223.5

(22) Date of filing: 20.03.85

(51) Int. Cl.⁴: **G 01 N 33/545**
G 01 N 33/577
//G01N33/94

(30) Priority: 22.03.84 US 592323

(43) Date of publication of application:
02.10.85 Bulletin 85/40

(84) Designated Contracting States:
AT BE CH DE FR GB IT LI LU NL SE

(71) Applicant: GENETIC DIAGNOSTIC CORPORATION
160 Community Drive
Great Neck, N.Y. 11021(US)

(72) Inventor: Deutsch, Alice
899 Broadway
New York, N.Y. 10003(US)

(74) Representative: Eggert, Hans-Gunther, Dr.
Räderscheidtstrasse 1
D-5000 Köln 41(DE)

(54) Automated immunoassay.

(57) A method is described for performing immunoassays on a continuously reusable short cycle device. The immunoassays are automated and the samples are serially analyzed with the samples and various liquids passing serially through various chambers and tubings. The method comprises mixing a sample of unknown amount of antigen with a predetermined amount of labeled antibody, and bringing this mixture into contact with a mass of antigen that has been immobilized on a tube thereby to bind at least part of the mixed labeled antibody., Then a substrate which changes in the presence of the label on the antibody is passed through the tube and the extent of signal is measured. Thereafter the tube is regenerated to break the antigen-antibody bond so that it is ready for the next cycle.

_ I _

This invention relates to an improved method for immunoassay in which 1) a short-cycle time, hence more rapid analysis, is achieved, 2) a tubing coated with antigen can be regenerated and reused indefinitely, and 3) the entire operation is automated.

## Background

Immunoassay techniques are based upon formation of a complex between the antigenic substance being assayed and an antibody or antibodies. Either the antigen or antibody can be labeled by a radioactive element, an enzyme, or a fluorescent element. This label permits detection and measurement of the antigen after separation of the complexed labeled antigen or antibody from uncomplexed labeled antigen or antibody.

At the present time, most immunoassays are rather slow, labor-intensive procedures. They involve multiple pipetting steps, several incubation periods and washing steps. The assays are done in batch-wise fashion. All samples are added at the same time, incubated at the same time, washed at the same time, etc., and all reagents are discarded at the end of the assay.

Patent 3,896,217 describes an automated immunoassay which uses regenerated immobilized antibody and labeled antigen, and the assays are done in a sequential fashion. However, there is a relatively low limit on the number of cycles through which the regenerated immobilized antibody can be used while still maintaining assay reliability.

-2-

It is accordingly an object of the present invention to provide an immunoassay procedure which permits an immobilized reagent to be regenerated and reused reliably in many cycles.

It is a further object of the invention to provide an immunoassay procedure which can be run through a piece of equipment in sequence.

These and other objects and advantages are realized in accordance with the present invention pursuant to which there is provided an immunoassay procedure wherein a known antigen is immobilized on a support; a labeled antibody specific to the antigen is contacted with a test sample which might contain the antigen, to permit complexing; the labeled antibody-sample mass is contacted with the immobilized antigen to effect binding; the immobilized antigen is thereafter contacted with a liquid substrate responsive to the label; and the liquid substrate is assayed to determine the extent to which it has been acted upon by the immobilized antigen and labeled antibody complex which is an index of the amount of antigen in the test sample.

In accordance with another aspect of the invention the immobilized antigen which is complexed is regenerated to release the complex and the cycle can then be repeated with a new test sample. The process can be carried out on a piece of equipment through which solutions flow in sequence in plug flow so that numerous tests can be reliably conducted rapidly and inexpensively.

By virtue of the fact that the antigen is re-used, if it is non-proteinaceous, there is no concern about its becoming denatured, which would be a problem if an antibody were the immobilized material. The reusable antigen can actually be held to the immobile support by a protein link and such protein link can even denature upon repeated use. So long as the antigen is still held, however, such denaturation poses no problem. Alternatively, the antigen can be linked directly to the immobile support without loss of antigenicity.

It is noted that the reading is of a liquid substrate which is uncontaminated by eluant, thereby giving more reliable readings.

The immobilizing support can be any of those conventionally employed, e.g., cellulose or other carbohydrate derivatives, but it is preferably plastic and particularly preferably nylon, which optionally is hydrolyzed as described in detail in Application Serial No. filed                , now pending, (Genetic Diagnostics 204) the disclosure of which is incorporated herein by reference. Thereby the antigen can be held more securely.

The antigen can be any of those conventionally employed in immunoassays, e.g., any of those disclosed in Application Serial No. 401,460, filed July 26, 1982, now pending, the disclosure of which is incorporated herein by reference. The invention is especially useful when working with small antigens, of which a representative member is digoxin. The antigen can be immobilized, i.e., joined to the solid support, in any conventional way. For example, the support can be contacted with a substance such as a modified antigen which readily adheres thereto. Alternatively, the bovine serum albumin (BSA) can first be contacted with the antigen which complexes with the BSA, and then the BSA-antigen complex is added to the support thereby becoming fixed to

-4-

the solid support so securely that it will not become
separated even after many cycles of contact with the liquids
involved in the subsequent assays.

The solid support is preferably in the form of a nylon
tube carrying the antigen on one or both surfaces,
preferably on its inside surface, so that reagent liquids
can later be caused to flow through the tube, the tube
length and the temperatures and speeds of flow of the
liquids being designed to afford predetermined reaction
times.

The test sample will be tested for its content of that
antigen which is immobilized on the support, in this case
digoxin. It will be contacted with a predetermined quantity
of an antibody for the test antigen, preferably a monoclonal
antibody. Such an antibody is disclosed in aforementioned
Application Serial No. 401,460. Any antigen in the sample
will bind to the antibody, thereby removing some antibody
from complexing or binding during the subsequent contact
with immobilized antigen. How much antibody has been
pre-complexed or bound is reflected by the activity of the
subsequent complex on the solid support.

The antibody, prior to contact with the test sample,
carries a label such as a fluorescent radical or, more
preferably, an enzyme. Accordingly, antigen-antibody
complex will carry that label as well.

The mixture of sample antigen and labeled antibody is
then contacted with the immobilized antigen, thereby fixing
to the immobilized antigen on the nylon tube whatever
antibody has not already been bound by the sample antigen.

This necessarily links the enzyme label to the nylon tube as well, the amount of label being correlated to the amount of antigen in the test sample.

The nylon tube is then contacted with a substrate responsive to the label, e.g., if the label is a β-galactosidase enzyme, then the substrate contains an ester which upon deesterification yields a fluorescent or colorimetric product. The substrate is removed from the nylon tube and is then assayed, fluorimetrically or spectrophotometrically.

Thereafter, the nylon tube is contacted with a liquid which will break the bond between the immobilized antigen and antibody, washing away the antibody and leaving regenerated immobilized antigen available for the next cycle. Suitable regenerants, i.e., desorbing agents, are known, e.g., sodium thiocyanate, potassium iodide, concentrated salts, etc..

The process can be carried out using a commercially available piece of equipment designed to permit sequential flow of numerous liquids, e.g., the Technicon Sequential Multiple Analyzer.

The invention will be further described with reference to the accompanying drawings wherein:

Fig. 1 is a schematic flow sheet of one specific process in accordance with the invention, and

Fig. 2 is a standard curve showing the relation between the amount of fluorescent product released from the substrate (MUG) due to reaction with the enzyme of the enzymatically labeled monoclonal antibody bound to antigen (digoxin) on the nylon tube, after contact of the labeled antibody with sample antigen, and different amounts of sample antigen.

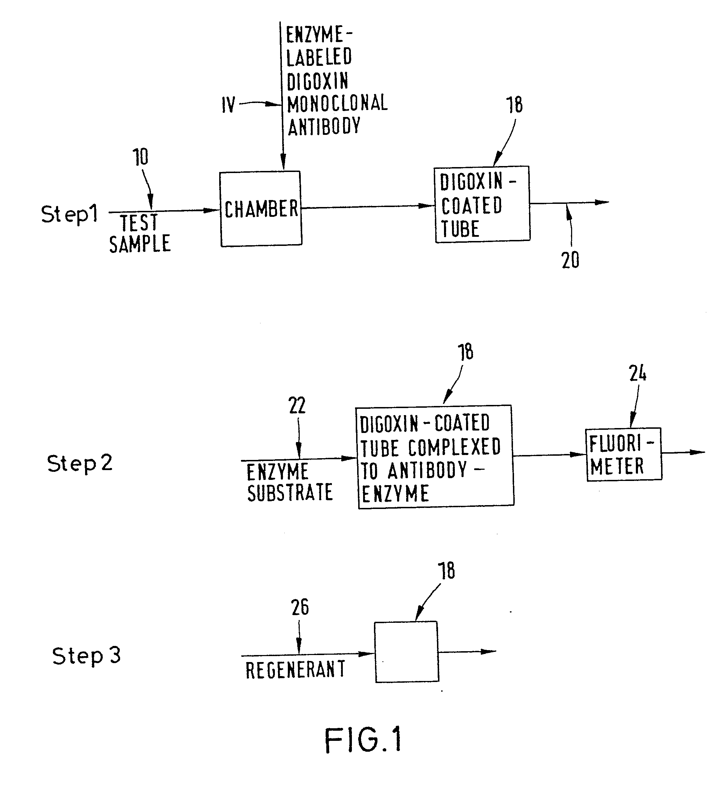

Referring now more particularly to Fig. 1, in the first step, a test sample 10 and a monoclonal antibody for digoxin 12, the antibody carrying a β-galactosidase enzyme, are mixed in a chamber 14 and, after some binding has taken place, the mixture passes at 16 into and through a narrow tube 18 of hydrolyzed nylon covalently coupled on its inside to BSA which is complexed with digoxin. After a predetermined contact time, waste liquid leaves the tube 18 at 20.

In the second step a predetermined quantity of a substrate 22 is passed through the tube 18 and then into a fluorimeter 24 where it is assayed. Specifically, the substrate 22 is one which changes its fluorescence upon being acted upon by β-galactosidase so that the amount of fluorescence observed at 24 will be an index of the amount of β-galactosidase on tube 18. This completes the assay.

In the third step a regenerating liquid or eluant 26 is passed through tube 18 to break the bonds between immobilized antigen and antibody-enzyme conjugate. Thereafter the tube is ready for the next cycle.

It is possible to include one or more washing steps between the illustrated steps to better isolate the steps from one another.

-7-

The invention will now be illustratively described in the following example wherein all parts are by weight unless otherwise specified.

Example I

The following tubings (available from Alpkem Corp.) were connected: A = 8.1 inches standard manifold pump tubing (flow rate = 0.6 ml/min) to B = 14.5 inches clear auto analyzer standard tubing to C=a 1.55 ml glass phasing coil to D = 12.3 inches modified nylon tubing to E = 10 inches of clear auto analyzer standard tubing. The tubing A was connected to an automatic manifold pump (Alpkem Corporation). The phasing coil (C) and modified nylon tubing (D) sat in a 37°C water bath. The dwell times were as follows: A + B = 2.28 minutes, C = 2.55 minutes, D = 2.05 minutes and E = 0.55 minute giving a total assay time of 7.43 minutes or 7.83 minutes if 0.3 minute is allowed for one air bubble between each reagent. The nylon tubing was 1/8 inch nylon pressure tubing obtained from Ain Plastics. Digoxin-bovine serum albumin from Immunotech was conjugated to the inside of the nylon tubing using a patent-submitted protocol.

The following solutions were aspirated into the above tubing system in the following order: 1.2 ml 3 M sodium thiocyanate, 1.2 ml 0.1% bovine serum albumin (BSA) in phosphate buffered saline (0.15 M sodium chloride in 20mM sodium phosphate buffer, pH 7.2) (PBS), 0.5 ml monoclonal antibody to digoxin conjugated to β-galactosidase plus 0.1 ml serum containing 1 ng/ml digoxin mixed together just before aspirating, 1.2 ml BSA in PBS, and 0.6 ml of 100 uM methyl umbelliferyl-β-D-galactoside (MUG). (All chemical reagents were obtained from Sigma except the β-galactosidase which was from Boehringer Mannheim.)

The monoclonal antibody was made as described in Application Serial No. 401,460, filed July 26, 1982, now pending. The monoclonal antibody β-galactosidase conjugate was made using the method of Deelder and DeWater (1981) J. Histochem. Cytochem. 29:1273-1280.

After all the above solutions went through the tubing system, the fluorescence of the MUG was determined in a Perkin-Elmer Fluorimeter No. 650-105.

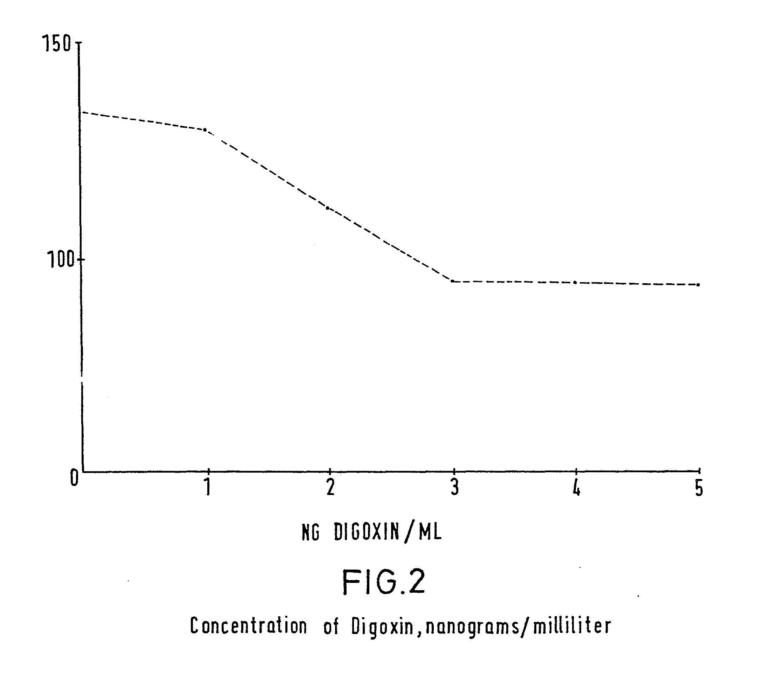

The cycle was repeated using different amounts of digoxin in serum. The results of a standard curve are shown in Fig. 2. The curve shows that as the amount of digoxin increases, the fluorescent signal decreases. The steepest part of the standard curve is between 1 and 3 ng/ml digoxin, which is the range needed for therapeutic monitoring. The results show that this automated tubing system can be used to measure digoxin in serum samples. Table I shows the results of reusing the automated tubing system. The assays could be run through the same digoxin-BSA tubing without detrimental loss in steepness of slope or straightness of standard curve.

## TABLE I

| Cycle No. | Fluorescence of 2 ng/ml Sample | Slope | Straight Line Correlation (0-3) |
|-----------|-------------------------------|-------|-------------------------------|
| 1 | 43 | -13.4 | 0.97 |
| 23 | 68 | -13.6 | 0.99 |
| 48 | 76 | -16.1 | 0.95 |

It is understood that the specification and examples are illustrative but not limitative of the present invention and that other embodiments within the spirit and scope of the invention will suggest themselves to those skilled in the art.

Claims

1. A tube covalently linked to a chemical which is an antigen.

2. A tube covalently linked to a protein which is complexed with an antigen.

3. A tube according to claim 2, wherein the protein is bovine serum albumin.

4. A tube according to claim 2, wherein the tube comprises hydrolyzed nylon.

5. A tube according to claim 2, wherein the antigen is digoxin.

6. A process for conducting an immunoassay comprising contacting with a labeled antibody a test sample which is to be assayed for an antigen specific to the antibody so as to permit reaction between the antibody and any antigen in the sample, contacting that reaction mass with a solid support having that antigen immobilized thereon so as to permit any available antibody to react with the immobilized antigen which thereby binds the label to the support, contacting the label-carrying support with a substrate responsive to the label, and assaying the substrate.

7. The process according to claim 6, including the further step of contacting the support with a regenerant so as to break any bonds between the immobilized antigen and antibody, thereby to free the antigen for subsequent reuse.

8. The process according to claim 7, including the reuse of the support.

9. The process according to claim 6, wherein the solid support comprises a tube to which the antigen is held by a protein.

10. The process according to claim 6, wherein the solid support comprises a tube to which the antigen is coupled directly.

11. The process according to claim 9, wherein the tube comprises hydrolyzed nylon.

12. The process according to claim 10, wherein the tube comprises hydrolyzed nylon.

13. The process according to claim 9, wherein the protein comprises bovine serum albumin.

14. The process according to claim 6, wherein the antibody is a monoclonal antibody.

15. The process according to claim 11, wherein the protein comprises bovine serum albumin and the antibody is a monoclonal antibody, the liquids being successively passed through the tube.

FIG.1

FIG.2

Concentration of Digoxin, nanograms/milliliter

# European Patent Office

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| D,X | EP-A-0 101 912 (GENETIC DIAGNOSTIC CORPORATION) * The whole document * | 1-6,9-14 | G 01 N 33/545 G 01 N 33/577// G 01 N 33/94 |
| Y | | 7,8,15 | |
| X | EP-A-0 079 466 (MAX-PLANCK-GESELLSCHAFT ZUR FÖRDERUNG DER WISSENSCHAFTEN e.V.) * Abstract; claims 1-6 * | 1 | |
| Y | | 2-15 | |
| X | INTERNATIONAL JOURNAL OF APPLIED RADIATION AND ISOTOPES, vol. 35, no. 1, January 1984, pages 49-54, Pergamon Press Ltd., Oxford, GB; G.L.ZUCCHINI et al.: "A procedure for the activation of a solid phase (Nylon 6) for bioanalytical purposes" * Introduction" & "Application" * | 1 | TECHNICAL FIELDS SEARCHED (Int. Cl.4) G 01 N |
| Y | IDEM. | 2-15 | |
| Y | EP-A-0 094 770 (BECTON, DICKINSON & COMPANY) * Abstract; claims 1,9 * | 1,7,8, 15 | |

-/-

The present search report has been drawn up for all claims

| Place of search | Date of completion of the search | Examiner |
|---|---|---|
| THE HAGUE | 21-06-1985 | OSBORNE H.H. |

**European Patent Office**

**EUROPEAN SEARCH REPORT**

Page 2

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| Y | WO-A-8 301 119 (COMMONWEALTH SERUM LABORATORIES COMMISSION) * Claim 12 * | 1,7,8, 15 | |

----

TECHNICAL FIELDS SEARCHED (Int. Cl.4)

The present search report has been drawn up for all claims

| Place of search | Date of completion of the search | Examiner |
|---|---|---|
| THE HAGUE | 21-06-1985 | OSBORNE H.H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82